(19) Europäisches Patentamt / European Patent Office / Office européen des brevets

(11) **EP 3 121 745 A1**

(12) **EUROPEAN PATENT APPLICATION**

(43) Date of publication:
**25.01.2017 Bulletin 2017/04**

(51) Int Cl.:
***G06F 19/00*** *(2011.01)*

(21) Application number: **16170131.3**

(22) Date of filing: **18.05.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **21.05.2015 JP 2015103371**

(71) Applicant: **SUMITOMO HEAVY INDUSTRIES, LTD. Tokyo 141-6025 (JP)**

(72) Inventor: **ICHISHIMA, Daiji Yokosuka-shi, Kanagawa 237-8555 (JP)**

(74) Representative: **Louis Pöhlau Lohrentz Patentanwälte Postfach 30 55 90014 Nürnberg (DE)**

(54) **SIMULATION METHOD, SIMULATION PROGRAM, AND SIMULATION DEVICE**

(57)     Disclosed is a method of performing a simulation by applying renormalization transformation in which a velocity distribution of grains is maintained. A renormalization transformation process is performed with respect to a granular system S which is a simulation target based on a renormalization factor $\alpha$ depending on the number of renormalizations. A position vector and a momentum vector of a grain of a renormalized granular system S' are calculated by executing molecular dynamics calculation with respect to the renormalized granular system S'. An interaction potential $\phi$ between the grains of the granular system S is expressed as $\phi (r) = \varepsilon f((r-r_0)/\sigma)$, where $\varepsilon$ represents an interaction coefficient having a dimension of energy, f represents a non-dimensional function, $r_0$ and $\sigma$ represent parameters characterizing a grain, and r represents an inter-grain distance. When a dimensionality of a space of the granular system S is represented as d, by applying transformation laws expressed as $N' = N/\alpha^d$, $m' = m\alpha^d$, $\varepsilon' = \varepsilon\alpha^d$, $r_0' = \alpha r_0$, and $\sigma' = \alpha\sigma$, the molecular dynamics calculation is executed based on an interaction potential of the renormalized granular system S' expressed as $\phi' (r) = \varepsilon' f ((r-r_0')/\sigma')$.

## FIG. 1

START

EXECUTE RENORMALIZATION TRANSFORMATION PROCESS WITH RESPECT TO GRANULAR SYSTEM S WHICH IS SIMULATION TARGET TO DEFINE RENORMALIZED GRANULAR SYSTEM S' — S1

SET INITIAL CONDITIONS OF SIMULATION — S2

EXECUTE MOLECULAR DYNAMICS CALCULATION WITH RESPECT TO RENORMALIZED GRANULAR SYSTEM S' TO CALCULATE POSITION VECTOR q' AND MOMENTUM VECTOR p' OF GRAIN — S3

OUTPUT SIMULATION RESULT — S4

END

EP 3 121 745 A1

**Description**

BACKGROUND OF THE INVENTION

Incorporation by Reference

Priority is claimed to Japanese Patent Application No. 2015-103371, filed May 21, 2015, the entire content of which is incorporated herein by reference.

Field of the Invention

The present invention relates to a simulation method, a simulation program, and a simulation device using molecular dynamics having an application with a renormalization group.

Description of the Related Art

Computer simulations using molecular dynamics are performed. In molecular dynamics, a motion equation of grains that form a system which is a simulation target is numerically solved. If the number of grains included in a system which is a simulation target increases, the amount of necessary calculation increases. The number of grains of a system capable of being simulated by a computation of existing computers is normally about several hundreds of thousands of pieces.

Japanese Patent No. 524168 discloses a simulation method using a renormalization transformation technique in order to reduce the amount of necessary calculation. Hereinafter, the renormalization transformation technique disclosed in Japanese Patent No. 524168 will be described.

The number of grains of a granular system S which is a simulation target is represented as N, the mass of each grain is represented as m, and an interaction potential between grains is represented as $\phi(r)$. Here, r represents an inter-grain distance. The interaction potential $\phi(r)$ is expressed as a product of an interaction coefficient $\varepsilon$ and a function $f(r)$. The interaction coefficient $\varepsilon$ represents the intensity of interaction, and has a dimension of energy. The function $f(r)$ represents dependency on an inter-grain distance, which is non-dimensional.

A first renormalization factor $\alpha$, a second renormalization factor $\gamma$, and a third renormalization factor $\delta$ are determined. The first renormalization factor $\alpha$ is larger than 1. The second renormalization factor $\gamma$ is equal to or larger than 0, and is equal to or smaller than a space dimensionality d. The third renormalization factor $\delta$ is equal to or greater than 0. When the number of renormalizations is represented as n, the first renormalization factor $\alpha$ is expressed as $\alpha=2n$.

The number of grains of a granular system S' which is renormalization-transformed using a renormalization technique is represented as N', the mass of each grain is represented as m', and an interaction coefficient is represented as $\varepsilon'$. The number of grains N' of the renormalization-transformed granular system S', the mass m', and the interaction coefficient $\varepsilon'$ are calculated using the following transformation equations.

$$N' = N / \alpha^d$$

$$m' = m\alpha^{\delta-\gamma}$$

$$\varepsilon' = \varepsilon\alpha^{\gamma}$$

Molecular dynamics calculation is performed with respect to the renormalization-transformed granular system S'. A position vector of each grain obtained by the molecular dynamics calculation is represented as q', and a momentum vector thereof is represented as p'. A position vector q and a momentum vector p of each grain of the granular system S may be calculated using the following equations.

$$q = q'\alpha$$

$$p = p' / \alpha^{\delta} / 2$$

Problems to be Solved by the Invention

A Maxwell velocity distribution law $f_{max}(v')$ of the renormalization-transformed granular system S' is expressed as the following equation.

$$f_{max}(v') = \exp[(-m'/2k_BT)v'^2]$$

Here, v' represents a velocity of each grain of the renormalization-transformed granular system S', $k_B$ represents Boltzmann's constant, and T represents a temperature of the granular system S'.
The above-mentioned Maxwell velocity distribution law $f_{max}(v')$ is rewritten as the following equation using the mass m of each grain of the original granular system S.

$$F_{max}(v') = \exp[(-m/2k_BT)(v'\alpha^{(\delta-\gamma)/2})^2]$$

The above equation means that a standard deviation of a velocity distribution of grains of the renormalization-transformed granular system S' is $1/\alpha(\delta-\gamma)/2$ times a standard deviation of a velocity distribution of grains of the original granular system S. That is, in the renormalization-transformed granular system S', the velocity distribution of the grains of the original granular system S is not reproduced. If the number of renormalizations n increases, the velocity distribution of the grains of the renormalization-transformed granular system S' deviates further from the velocity distribution of the grains of the original granular system S.
If the velocity distribution of the renormalization-transformed granular system S' and the velocity distribution of the original granular system S are greatly different from each other, the amount of evaporation, generation of droplets, elimination, or the like is not correctly reproduced.

SUMMARY OF THE INVENTION

An object of the invention is to provide a method for performing simulation by applying renormalization transformation in which a velocity distribution of grains is maintained. Another object of the invention is to provide a computer program for performing a simulation by applying renormalization transformation in which a velocity distribution of grains is maintained. Still another object of the invention is to provide a device that performs a simulation by applying renormalization transformation in which a velocity distribution of grains is maintained.
According to an aspect of the invention, there is provided a simulation method including: a process of performing a renormalization transformation process with respect to a granular system S which is a simulation target formed of a plurality of grains based on a renormalization factor $\alpha$ depending on the number of renormalizations; and a process of calculating a position vector and a momentum vector of a grain of a renormalized granular system S', by executing molecular dynamics calculation with respect to the renormalized granular system S'. When an interaction potential $\phi$ between the grains of the granular system S is expressed as follows,

$$\phi(r) = \varepsilon \cdot f\left(\frac{r - r_0}{\sigma}\right)$$

where $\varepsilon$ represents an interaction coefficient having a dimensionality of energy, f represents a non-dimensional function, $r_0$ and $\sigma$ represent parameters characterizing a grain, and r represents an inter-grain distance, and when a dimension of a space of the granular system S is represented as d, by applying transformation laws expressed as follows,

$$N' = \frac{N}{\alpha^d}$$

$$m' = m \cdot \alpha^d$$

$$\varepsilon' = \varepsilon \cdot \alpha^d$$

$$r_0' = \alpha \cdot r_0$$

$$\sigma' = \alpha \cdot \sigma$$

the molecular dynamics calculation is executed based on an interaction potential of the renormalized granular system S' expressed as follows:

$$\phi'(r) = \varepsilon' \cdot f\left(\frac{r - r_0'}{\sigma'}\right)$$

According to another aspect of the invention, there is provided a computer program that executes the above-described simulation method. According to still another aspect of the invention, there is provided a recording medium on which the above-described computer program is recorded. According to yet still another aspect of the invention, there is provided a simulation device that executes the above-described simulation method.

Effects of the Invention

According to the above-described simulation method, a velocity distribution of a renormalized granular system becomes the same as a velocity distribution of an original granular system.

BRIEF DESCRIPTION OF THE DRAWINGS

FIG. 1 is a flowchart of a simulation method according to an embodiment.
FIG. 2 is a conceptual diagram illustrating grains arranged in a one-dimensional pattern.
FIG. 3 is a graph illustrating a calculation result of Equation (21) and a numerical integration result of Equation (22) in a case where an interaction potential $\phi$ is a Lennard-Jones potential.
FIG. 4 is a graph illustrating a calculation result of Equation (21) and a numerical integration result of Equation (22) in a case where an interaction potential $\phi$ is a Morse potential.
FIGS. 5A to 5C are conceptual diagrams illustrating grains arranged in a two-dimensional lattice pattern, and an interaction between grains.
FIGS. 6A to 6D are diagrams illustrating simulation results using a simulation method according to an embodiment.
FIGS. 7A to 7D are diagrams illustrating simulation results using a simulation method according to a comparative example.
FIG. 8 is a block diagram of a simulation device according to an embodiment.

DESCRIPTION OF THE PREFERRED EMBODIMENTS

Molecular Dynamics applied to an embodiment of the invention will be briefly described. A granular system formed of N grains (for example, atoms) and having a Hamiltonian H expressed as the following equation will be described.

$$H = \sum_{j=1}^{N}\left[\frac{\vec{p}_j^2}{2m} + \sum_{i=j+1}^{N}\phi\left(|\vec{q}_i - \vec{q}_j|\right)\right] \cdots (4)$$

Here, m represents the mass of a grain, $\phi$ represents an interaction potential between grains, a vector $p_j$

represents a momentum vector of the grain, and a vector $q_j$ represents a position vector (position coordinates) of the grain.

By substituting the Hamiltonian H in a Hamiltonian canonical equation, the following motion equation with respect to a grain j is obtained.

$$\frac{d\vec{p}_j}{dt} = \sum_{i \neq j}^{N-1} \left[ \frac{\partial \phi\left(\left|\vec{q}_i - \vec{q}_j\right|\right)}{\partial \vec{q}_j} \right] \cdots (5)$$

$$\frac{d\vec{q}_j}{dt} = \frac{\vec{p}_j}{m} \cdots (6)$$

In molecular dynamics, by solving the motion equations expressed by Equation (5) and Equation (6) by numerical integration with respect to each grain that forms a granular system, the momentum vector $p_j$ and the position vector $q_j$ of each grain at each time point are obtained. In many cases, a Verlet algorithm is used in the numerical integration. The Verlet algorithm is described in page 175 of "Computational Physics", J.M. Thijssen (Cambridge University Press 1999), for example. Various physical quantities of a granular system maybe calculated based on a momentum vector and a position vector of each grain obtained through molecular dynamics calculation.

Next, molecular dynamics using a renormalization group technique (hereinafter, referred to as renormalization group molecular dynamics) will be conceptually described.

In the renormalization group molecular dynamics, a granular system S which is a simulation target is associated with a granular system S' (hereinafter, referred to as a renormalized granular system S') formed of grains smaller in number than grains of the granular system S. Then, the molecular dynamics calculation is executed with respect to the renormalized granular system S'. A calculation result with respect to the renormalized granular system S' is associated with the granular system S which is the simulation target. Thus, it is possible to reduce the amount of calculation, compared with a case where the molecular dynamics calculation is directly executed with respect to the granular system S which is the simulation target. A transformation law for associating physical quantities (for example, the number of grains, the mass of a grain, and the like) in the granular system S which is the simulation target with the physical quantity in the renormalized granular system S' is referred to as a renormalization transformation law.

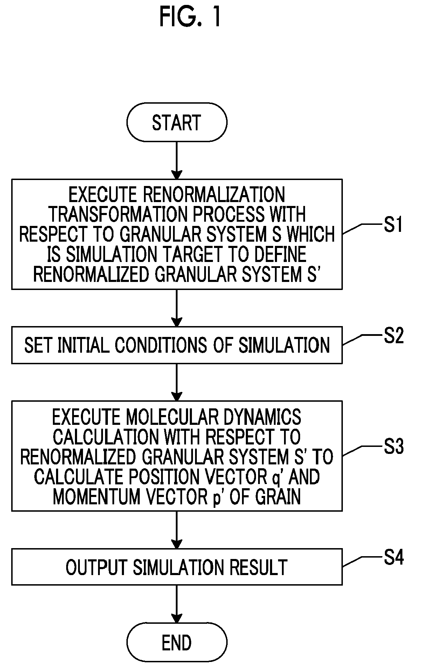

FIG. 1 shows a flowchart of a simulation method according to an embodiment. In step S1, a renormalization transformation process is executed based on the renormalization transformation law with respect to the granular system S which is the simulation target to define the renormalized granular system S'. In the granular system S which is the simulation target, an interaction potential between grains is expressed as the following equation.

$$\phi(r) = \varepsilon f\left(\frac{r - r_0}{\sigma}\right) \cdots (7)$$

Here, r represents an inter-grain distance, f represents a non-dimensional function, and $\varepsilon$, $r_0$, and $\sigma$ represent parameters characterizing a grain (for example, an atom or a molecule). $\varepsilon$ has a dimension of energy, and is called as an interaction coefficient. $r_0$ corresponds to a position where the interaction potential $\phi$ becomes a minimum. In an equilibrium state, the inter-grain distance is approximately the same as $r_0$.

In a case where the grains of the granular system S which is the simulation target are inert atoms, the Lennard-Jones potential may be applied as the interaction potential $\phi$. The Lennard-Jones potential is defined as the following equation, for example.

$$\phi(r) = \varepsilon \left[ \left(\frac{\sigma}{r}\right)^{12} - \left(\frac{\sigma}{r}\right)^6 \right] \cdots (8)$$

Equation (8) may be changed into the following equation.

$$\phi(r) = \varepsilon \left[ \left( \cfrac{1}{\cfrac{r-r_0}{\sigma} + \cfrac{r_0}{\sigma}} \right)^{12} - \left( \cfrac{1}{\cfrac{r-r_0}{\sigma} + \cfrac{r_0}{\sigma}} \right)^{6} \right] \cdots (9)$$

As understood from Equation (9), the Lennard-Jones potential is a function of $(r-r_0)/\sigma$, which may be expressed in the form of Equation (7).

In a case where the grains of the granular system S which is the simulation target are metallic atoms, the Morse potential maybe applied as the interaction potential $\phi$. The Morse potential may be defined as the following equation, for example.

$$\phi(r) = \varepsilon \left[ \exp\left( -2\frac{r-r_0}{\sigma} \right) - 2\exp\left( -\frac{r-r_0}{\sigma} \right) \right] \cdots (10)$$

The physical quantities N, m, $\varepsilon$, $r_0$, and $\sigma$ of the granular system S which is the simulation target are transformed into physical quantities N', m', $\varepsilon$', $r_0$', and $\sigma$' of the granular system S' which are respectively renormalized through the renormalization transformation process. In the renormalization transformation process of step S1, the following renormalization transformation law is applied.

$$N' = \frac{N}{\alpha^d}$$

$$m' = m \cdot \alpha^d$$

$$\varepsilon' = \varepsilon \cdot \alpha^d$$

$$r_0' = \alpha \cdot r_0$$

$$\sigma' = \alpha \cdot \sigma \cdots (11)$$

Here, d represents a dimensionality of a space where the granular system S which is the simulation target is arranged. $\alpha$ represents a renormalization factor depending on the number of times of renormalization. When the number of times of renormalization is n, the renormalization factor $\alpha$ is expressed as the following equation.

$$\alpha = 2^n \cdots (12)$$

The interaction potential $\phi$' of the renormalized granular system S' may be expressed as the following equation.

$$\phi'(r) = \varepsilon' f\left( \frac{r-r_0'}{\sigma'} \right) \cdots (13)$$

A Hamiltonian H' of the renormalized granular system S' may be expressed as the following equation, as described later in detail.

$$H' = \sum_{j=1}^{N'} \left[ \frac{\vec{p}_j'^2}{2m'} + \sum_{i=j+1}^{N'} \varepsilon' f\left( \frac{\left| \vec{q}_i - \vec{q}_j \right| - r_0'}{\sigma'} \right) \right] \cdots (14)$$

By applying the above-described renormalization transformation law, the number of grains becomes $1/\alpha^d$ times, and the mass of a grain becomes $\alpha^d$ times. Thus, the entire mass of the granular system S and the entire mass of the granular system S' are the same. Further, the inter-grain distance $r_0$ becomes $\alpha$ times. Thus, a dimension of the granular system S and a dimension of the renormalized granular system S' are the same. Since the entire mass of the granular system and the dimension thereof do not change before and after the renormalization transformation process, the density of the granular system is not also changed.

Then, in step S2, initial conditions of a simulation are set. The initial conditions include initial values of the position vector $q_j$ and the momentum vector $p_j$ of each grain. The momentum vector $p_j$ is set based on a temperature T' of the renormalized granular system S'. When the temperature of the granular system S which is the simulation target is represented as T, the following renormalization transformation is applied with respect to the temperature.

$$T' = T \cdot \alpha^d \cdots (15)$$

Then, in step S3, molecular dynamics calculation is executed with respect to the renormalized granular system S'. Specifically, the Hamiltonian H' of the renormalized granular system S' expressed as Equation (13) is substituted in the canonical equation to obtain a motion equation. The motion equation is expressed as the following equation.

$$\frac{d\vec{p}_i'}{dt'} = -\varepsilon' \sum_{j \neq i}^{N'} \frac{\partial}{\partial \vec{q}_i'} f\left( \frac{\left| \vec{q}_i' - \vec{q}_j' \right|}{\sigma'} \right)$$

$$\frac{d\vec{q}_i'}{dt'} = \frac{\vec{p}_i'}{m'} \cdots (16)$$

The motion equation is solved by numerical integration. Thus, time histories of a position vector q' and a momentum vector p' of each grain of the renormalized granular system S' are calculated.

The position vector q' and the momentum vector p' of each grain of the renormalized granular system S', and the position vector q and the momentum vector p of the granular system S which is the simulation target have the following relationship.

$$\vec{q}' = \vec{q}$$

$$\vec{p}' = \alpha^d \cdot \vec{p} \cdots (17)$$

In step S4, the simulation result is output. For example, the position vector q' and the momentum vector p' may be output as numerical values as they are, or may be displayed as an image obtained by imaging a distribution of plural grains of the granular system S' in a space based on the position vector q'. Further, by driving an actuator based on the simulation result, it is possible to apply a physical action to an object (as an observer).

Next, derivation of the Hamiltonian H' of the renormalized granular system S' will be described. In order to obtain the Hamiltonian H' of the renormalized granular system S', a part of integration of a partition function $Z(\beta)$ with respect to the granular system S may be executed to perform coarse graining with respect to a Hamiltonian, to thereby obtain the Hamiltonian H'.

The partition function $Z(\beta)$ with respect to a canonical ensemble having a constant number of grains is expressed as the following equation.

$$Z(\beta) = \int d\Gamma_N \exp\left( -\beta H(p, q) \right) \cdots (18)$$

$$\beta \equiv \frac{1}{k_B T}$$

Here, $d\Gamma_N$ represents a volume element in a phase space, which is expressed as the following equation.

$$d\Gamma_N = \frac{1}{W_N} \prod_{j=1}^{N} d\vec{p}_i \cdot d\vec{q}_i \equiv \frac{1}{W_N} D_p^N D_q^N \cdots (19)$$

$$D_p^N \equiv \prod_{j=1}^{N} d\vec{p}_i$$

$$D_q^N \equiv \prod_{j=1}^{N} d\vec{q}_i$$

$$W_N = N! h^{3N}$$

Here, h represents a Planck constant. $W_N$ is determined so that an intrinsic quantal sum of all states and integration over the phase space match each other.

First, coarse graining of an interaction potential between grains will be described, and then, coarse graining of a kinetic energy will be described. Subsequently, the renormalization transformation law is defined based on the coarse graining of the interaction potential and the coarse graining of the kinetic energy.

[Coarse graining of interaction potential between grains]

First, coarse graining of an interaction potential in a granular system where grains are arranged in a one-dimensional chain pattern will be described. Then, an interaction potential in a granular system where grains are arranged in a simple cubic lattice pattern will be described.

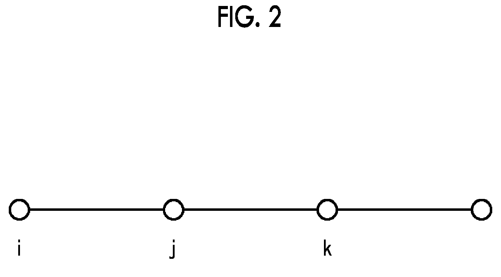

As shown in FIG. 2, a grain i, a grain j, and a grain k are sequentially arranged in a one-dimensional pattern. By writing an interaction relating to the grain j and executing integration with respect to position coordinates of the grain j positioned in the middle of the grain i and the grain k, it is possible to perform coarse graining of the interaction potential. First, in order to reflect contribution from a next-nearest or more distant grain, a potential moving method may be used. The potential moving method is described in "STATISTICAL PHYSICS Static, Dynamics and Renormalization", Chap. 14, World Scientific (1999) by Leo P. Kadanoff.

An interaction potential ϕ Tilda in which the contribution from the next-nearest or more distant grain is reflected may be expressed as the following equation.

$$\tilde{\phi}(r) = \phi(r) + \phi(r+a) + \phi(r+2a) + \bullet \bullet \bullet \cdots (20)$$

Here, a represents an inter-grain distance in an equilibrium state. The inter-grain distance a in the equilibrium state may be approximated to be equal to the distance $r_0$ where the interaction potential ϕ becomes minimum.

Since plural grains are arranged in a one-dimensional pattern, the position vector $q_j$ of the grain j may be expressed as a one-dimensional coordinate $q_j$. If the position of the grain j is expressed as $q_j$, a cage potential made by a nearest grain with respect to the grain j is expressed as the following equation.

$$\widetilde{\phi}(q_i - q_j) + \widetilde{\phi}(q_j - q_k)$$
$$= \widetilde{\phi}\left(\frac{q_i - q_k}{2} + \frac{q_i + q_k - 2q_j}{2}\right) + \widetilde{\phi}\left(\frac{q_i - q_k}{2} - \frac{q_i + q_k - 2q_j}{2}\right)$$
$$= \widetilde{\phi}\left(\frac{q_i - q_k}{2} + x_j\right) + \widetilde{\phi}\left(\frac{q_i - q_k}{2} - x_j\right)$$
$$= 2\left[\widetilde{\phi}\left(\frac{q_i - q_k}{2}\right) + \sum_{n=1}^{\infty}\frac{1}{2n!}\widetilde{\phi}^{(2n)}\left(\frac{q_i - q_k}{2}\right)x_j^{2n}\right]\cdots(21)$$
$$x_j = \frac{q_i + q_k - 2q_j}{2}$$

If integration is executed with respect to $q_j$ which is an integration variable, the following equation is obtained using Equation (21).

$$\int_{q_i + r_a}^{q_k - r_a} dq_j \exp\left[-\beta\{\widetilde{\phi}(q_i - q_j) + \widetilde{\phi}(q_j - q_k)\}\right]$$
$$= z(q_i - q_k)P(q_i - q_k)\cdots(21)$$

Here, $r_a$ represents the diameter of a grain, and $z(q_i - q_k)$ and $P(q_i - q_k)$ are expressed as the following equations.

$$P(q_i - q_k) = \exp\left[-2\beta\widetilde{\phi}\left(\frac{q_i - q_k}{2}\right)\right]\cdots(23)$$

$$z(q_i - q_k) = \int_{r_a}^{a - r_a} dx_j \exp\left[-2\beta\sum_{n=1}^{\infty}\frac{1}{2n!}\widetilde{\phi}^{(2n)}\left(\frac{q_i - q_k}{2}\right)x_j^{2n}\right]\cdots(24)$$

An integration region is limited to an inner region of the cage potential.

Then, $z(q_i - q_k)$ is specifically calculated. In a case where the interaction potential $\phi$ is the Lennard-Jones potential, $\phi^{(2n)}$ is expressed as the following equation.

$$\phi^{(2n)}(r) = \frac{4\varepsilon}{\sigma^{2n}}\left[\frac{(2n+11)!}{11!}\left(\frac{\sigma}{r}\right)^{2n+12} - \frac{(2n+5)!}{5!}\left(\frac{\sigma}{r}\right)^{2n+6}\right]\cdots(25)$$

In a case where the interaction potential $\phi$ is the Morse potential, $\phi^{(2n)}$ is expressed as the following equation.

$$\phi^{(2n)}(r) = \frac{\varepsilon}{\sigma^{2n}}\left[2^{2n}\exp\left(-2\frac{r - r_0}{\sigma}\right) - 2\exp\left(-\frac{r - r_0}{\sigma}\right)\right]\cdots(26)$$

Numerical integration is performed by substituting Equation (25) or Equation (26) in Equation (24). When substituting Equation (25) or Equation (26) in Equation (24), Equation (20) is used. In the numerical integration, it is assumed that "a" which appears in an integration range of Equation (24) is approximately equal to $r_0$.

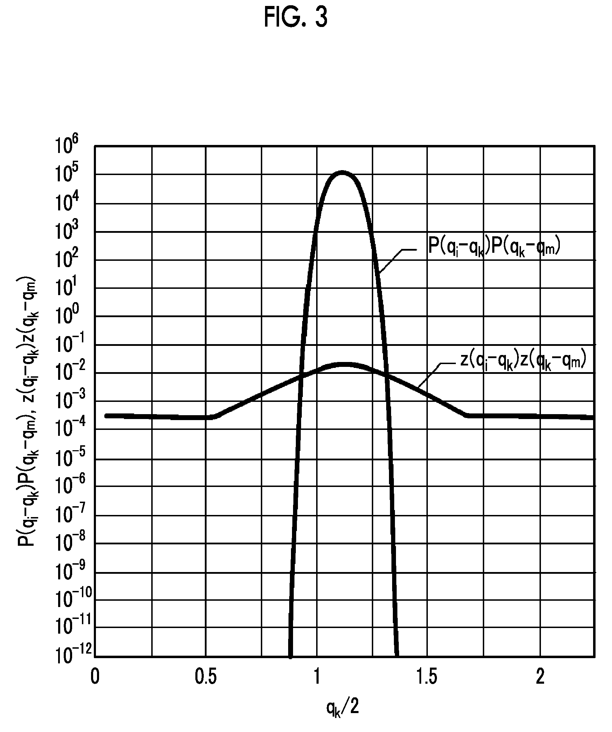

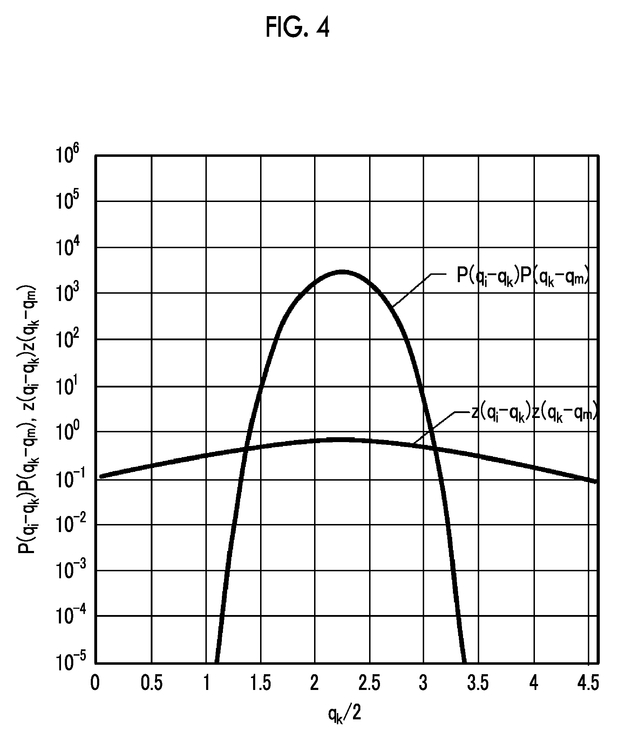

FIG. 3 shows a calculation result of Equation (23) and a numerical integration result of Equation (24) in a case where the interaction potential $\phi$ is the Lennard-Jones potential. FIG. 4 shows a calculation result of Equation (23) and a numerical integration result of Equation (24) in a case where the interaction potential $\phi$ is the Morse potential. In a case where a grain i, a grain j, a grain k, a grain 1, and a grain m are sequentially arranged in a one-dimensional pattern, a position coordinate of the grain k is expressed as $q_k$. A transverse axis in FIGS. 3 and 4 represents $q_k/2$, and a longitudinal axis represents $P(q_i - q_k)$ $P(q_k - q_m)$ and $z(q_i - q_k)$ $z(q_k - q_m)$ in a logarithmic scale. In the numerical value calculation in FIG. 3, it is assumed that $\varepsilon/k_BT=2$. and $r_0/\sigma=1.12$. In the numerical value calculation in FIG. 4, it is assumed that $\varepsilon/k_BT=2.0$ and $r_0/\sigma=2.24$.

In both cases where the interaction potential $\phi$ is the Lennard-Jones potential and where the interaction potential $\phi$ is the Morse potential, it can be understood that a change in z $(q_i$-$q_k)$ z $(q_k$-$q_m)$ is smoother than a change in P $(q_i$-$q_k)$ P $(q_k$-$q_m)$. Thus, z $(q_i$-$q_k)$ z $(q_k$-$q_m)$ may be nearly approximated as a constant with respect to P $(q_i$-$q_k)$ P $(q_k$-$q_m)$.

A probability $p(q_k)$ that the grain k is present in the position coordinate $q_k$ may be approximated as follows.

$$p(q_k) = \frac{z(q_i - q_k)z(q_k - q_m)P(q_i - q_k)P(q_k - q_m)}{\int_{q_i + r_a}^{q_m - r_a} dq_k z(q_i - q_k)z(q_k - q_m)P(q_i - q_k)P(q_k - q_m)}$$

$$\cong \frac{P(q_i - q_k)P(q_k - q_m)}{\int_{q_i + r_a}^{q_m - r_a} dq_k P(q_i - q_k)P(q_k - q_m)} \cdots (27)$$

Accordingly, the following equation is derived.

$$\int_{q_i + r_a}^{q_k - r_a} dq_j \exp\left[-\beta\left\{\tilde{\phi}(q_i - q_j) + \tilde{\phi}(q_j - q_k)\right\}\right]$$

$$\propto \exp\left[-2\beta\tilde{\phi}\left(\frac{q_i - q_k}{2}\right)\right] \cdots (28)$$

Hereinbefore, coarse graining of an interaction potential of a granular system in which plural grains are arranged in a one-dimensional pattern is described. An interaction potential of a multi-dimensional granular system may be realized by a potential moving method.

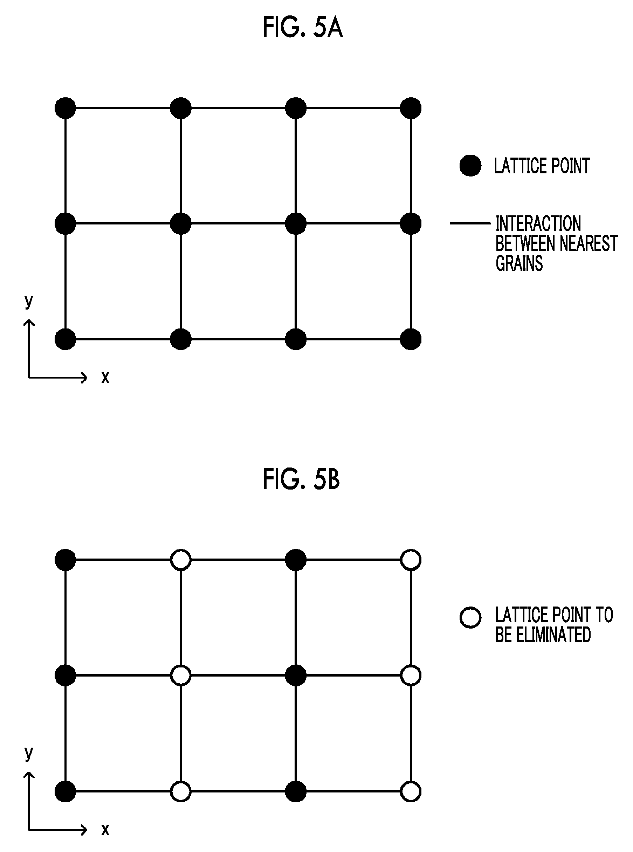

A potential moving method for returning a two-dimensional lattice to a one-dimensional lattice will be described with reference to FIGS. 5A to 5C.

As shown in FIG. 5A, grains are arranged at positions of lattice points of a two-dimensional square lattice. An interaction between nearest grains (nearest-neighbor-coupling) is indicated by a solid line. One direction where grains are arranged is defined as an x direction, and a direction orthogonal thereto is defined as a y direction.

As shown in FIG. 5B, it is considered that integration is executed with respect to displacements of grains (grains indicated by hollow circles) which are alternately arranged among grains arranged in the x direction. A grain which is an integration target (a grain to be eliminated) is referred to as an integration target grain.

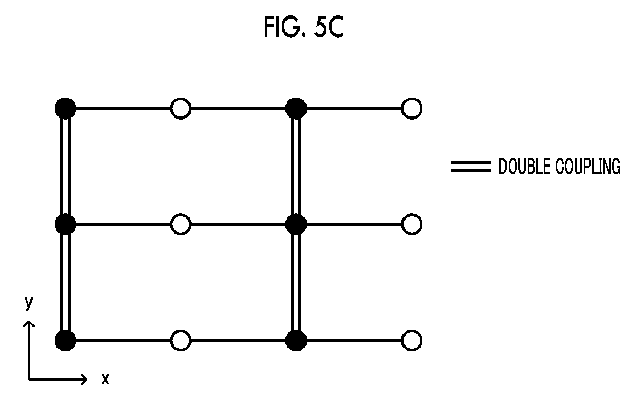

As shown in FIG. 5C, in the potential moving method, the nearest-neighbor-coupling of integration target grains is divided into grains which are adjacently arranged in the x direction. A grain interaction (double coupling) obtained by adding up divided interactions is indicated by a double-line. The double coupling indicated by the double-line has a strength two times the original nearest-neighbor-coupling. Using such a method, it is possible to transform a two-dimensional lattice into a one-dimensional chain. In the granular system of the one-dimensional chain, it is possible to perform coarse graining of an interaction potential by the method described with reference to FIG. 2. In a case where a granular system which is a simulation target forms a three-dimensional lattice, a procedure of transforming a two-dimensional lattice into a one-dimensional chain may be repeated in respect to three directions of the x direction, the y direction, and the z direction. In this way, it is possible to perform coarse graining of a granular system that forms a multi-dimensional lattice.

Coarse graining of an interaction potential of a granular system that forms a multi-dimensional (dimensionality d) lattice is expressed as the following equation.

$$\int D_q^N \exp\left(-\beta\sum_{j=1}^{N}\sum_{i=j+1}^{N}\phi(|\bar{q}_i - \bar{q}_j|)\right)$$

$$\propto \int D_q^{N'} \exp\left(-\beta \sum_{<i,j>}^{N'} 2^d \tilde{\phi}\left(\frac{|\vec{q}_i - \vec{q}_j|}{2}\right)\right) \cdots (29)$$

$$N' = \frac{N}{2^d}$$

Here, <i, j> means that a sum is taken between nearest lattices.

If Equation 29 is changed with respect to the sum of all interactions, the following equation is obtained.

$$\int D_q^N \exp\left(-\beta \sum_{j=1}^{N} \sum_{i=j+1}^{N} \phi(|\vec{q}_i - \vec{q}_j|)\right)$$

$$\propto \int D_q^{N'} \exp\left(-\beta \sum_{j=1}^{N'} \sum_{i=j-1}^{N'} 2^d \phi\left(\frac{|\vec{q}_i - \vec{q}_j|}{2}\right)\right) \cdots (30)$$

[Coarse graining of kinetic energy]

Next, coarse graining of a kinetic energy will be described. Integration may be easily executed with respect to the kinetic energy, and accordingly, the following equation is derived.

$$\int D_p^N \exp\left(-\beta \sum_{j=1}^{N} \frac{\vec{p}_j^2}{2m}\right) \propto \int D_p^{N'} \exp\left(-\beta \sum_{j=1}^{N'} \frac{\vec{p}_j^2}{2m}\right) \cdots (31)$$

In derivation of Equation (31), the following equation is used. Here, a momentum vector $p_j^2$ means an inner product of the vector.

$$\int \cdots \int d\vec{p}_i d\vec{p}_j d\vec{p}_k \cdots \exp\left(\cdots -\beta \frac{\vec{p}_i^2}{2m} - \beta \frac{\vec{p}_j^2}{2m} - \beta \frac{\vec{p}_k^2}{2m} \cdots\right)$$

$$= \sqrt{\frac{2m}{\beta}} \int \cdots \int d\vec{p}_i d\vec{p}_k \cdots \exp\left(\cdots -\beta \frac{\vec{p}_i^2}{2m} - \beta \frac{\vec{p}_k^2}{2m} \cdots\right) \cdots (32)$$

[Derivation of renormalization transformation law]

Next, a renormalization transformation law derived from coarse graining of the above-described interaction potential and coarse graining of a kinetic energy will be described.

By substituting Equation (30) and Equation (31) in Equation (18) to eliminate coefficients which do not affect a result, the following equation is obtained.

$$Z(\beta) = \int d\Gamma_{N'} \exp\left[-\beta \sum_{j=1}^{N'}\left\{\frac{\vec{p}_j^2}{2m} + \sum_{i=j+1}^{N'} 2^d \varepsilon f\left(\frac{|\vec{q}_i - \vec{q}_j| - 2r_0}{2\sigma}\right)\right\}\right] \cdots (33)$$

From Equation (33), a Hamiltonian H' (Hamiltonian of the renormalized granular system S') which is subject to coarse graining is expressed as the following equation.

$$H' = \sum_{j=1}^{N'} \left\{ \frac{\vec{p}_j^2}{2m} + \sum_{i=j+1}^{N'} 2^d \varepsilon f\left( \frac{|\vec{q}_i - \vec{q}_j| - 2r_0}{2\sigma} \right) \right\} \cdots (34)$$

$$N' = \frac{N}{2^d}$$

A list of coupling constants when performing coarse graining of the Hamiltonian is represented as K. The list K of the coupling constants is expressed as follows.

$$K = (m, \varepsilon, \sigma, r_0) \cdots (35)$$

The renormalization transformation R is defined as follows.

$$K' = R(K) = (2^d m, 2^d \varepsilon, 2\sigma, 2r_0) \cdots (36)$$

A list $K_n$ of coupling coefficients after renormalization transformation is executed n times is expressed as the following equation.

$$K_n = R \circ \cdots \circ R(K) = (\alpha^d m, \alpha^d \varepsilon, \alpha\sigma, \alpha r_0) \cdots (37)$$

$$\alpha = 2^n$$

Accordingly, a Hamiltonian Hn after renormalization transformation is performed n times is expressed as the following equation.

$$H_n = R \circ \cdots \circ RH$$
$$= \sum_{j=1}^{\frac{N}{\alpha^d}} \left\{ \frac{\vec{p}_j'^2}{2\alpha^d m} + \sum_{i=j+1}^{\frac{N}{\alpha^d}} \alpha^d \varepsilon f\left( \frac{|\vec{q}_i - \vec{q}_j| - r_0\alpha}{\sigma\alpha} \right) \right\} \cdots (38)$$

Here, the momentum vector pj' in the renormalized granular system S' is expressed as the following equation.

$$\vec{p}_j' = \alpha^d \vec{p}_j \cdots (39)$$

The renormalization transformation law shown in Equation (11) and the Hamiltonian H' of the renormalized granular system S' shown in Equation (14) are derived from Equation (38).
Next, excellent effects of the simulation method according to the embodiment will be described. A Maxwell's velocity distribution law in the renormalized granular system S' is expressed as the following equation.

$$f_{\max}(v') = \exp\left( -\frac{m'}{2k_B T'} v'^2 \right) \cdots (40)$$

By substituting an equation relating to m' of Equation (11) and Equation (15) in Equation (40), the following equation is obtained.

$$f_{\max}(v') = \exp\left(-\frac{m}{2k_B T}v'^2\right)\cdots(41)$$

As understood from Equation (41), a velocity distribution of the renormalized granular system S' is the same as a velocity distribution of the original granular system S before renormalization. Thus, it is possible to enhance reproducibility of a phenomenon relating to a behavior of a grain on an interface of the granular system S, for example, the amount of evaporation, generation and elimination of droplets, or the like.

Further, in the simulation method according to the embodiment, the number of grains N is transformed into $1/\alpha^d$ times, and the inter-grain distance $r_0$ in the equilibrium state is transformed into $\alpha$ times. Thus, the dimensions of the granular systems before and after renormalization transformation do not change. Further, the number of grains N is transformed into $1/\alpha^d$ times, and the mass of a grain is transformed into $\alpha^d$ times. Thus, the densities of granular systems before and after renormalization transformation do not change.

Since the dimensions and densities of the granular systems do not change, it is possible to use the simulation method according to the above-described embodiment and a simulation method such as a finite element method for approximating a continuous body in parallel. For example, in a system where an elastic body and a liquid are in contact with each other, it is possible to analyze the elastic body by the finite element method, and to analyze the liquid by the molecular dynamics calculation according to the above-described embodiment.

Next, a result obtained by performing a three-dimensional dam break simulation using the simulation method according to the above-described embodiment will be described.

First, conditions of the simulation will be described. A used interaction potential is a Lennard-Jones potential. Specifically, the interaction potential is expressed as the following equation.

$$\phi(r) = 4\varepsilon\left[\left(\frac{\sigma}{r}\right)^{12} - \left(\frac{\sigma}{r}\right)^{6}\right]\cdots(42)$$

Here, the function f in Equation (7) is expressed as the following equation.

$$f\left(\frac{r-r_0}{\sigma}\right) = 4\left[\left(\frac{\sigma}{r}\right)^{12} - \left(\frac{\sigma}{r}\right)^{6}\right] = 4\left[\left(\frac{1}{\frac{r-r_0}{\sigma}+\frac{r_0}{\sigma}}\right)^{12} - \left(\frac{1}{\frac{r-r_0}{\sigma}+\frac{r_0}{\sigma}}\right)^{6}\right]\cdots(43)$$

Coupling constants $\varepsilon$, $\sigma$, and $r_0$ are set as the following values. The values correspond to values of argon (Ar) atoms.

$$\varepsilon = 119.8[K]$$

$$\sigma = 0.3405[nm]$$

$$r_0 = 2^{\frac{1}{6}}\sigma\cdots(44)$$

An appearance of the dam before break is a rectangular body having a height H, a lateral width L, and a thickness D. At time t=0, a behavior of a granular system after one surface orthogonal to a lateral width direction is broken is simulated. A lateral width of the dam after break is 2L. As the height H, the lateral width L, and the thickness D, the following values are employed.

$$H = 0.052[m]$$

$$L = 0.052[m]$$

$$D = 0.0057[m] \cdots (45)$$

Since the dimension of the granular system does not change due to renormalization transformation, a height H', a lateral width L', and a thickness D' of an appearance of a dam of the renormalized granular system S' before break are the same as the height H, the lateral width L, and the thickness D of the original granular system S, respectively.

As simulation conditions, a gravitational acceleration g' after renormalization transformation, the number of grains N' after renormalization transformation, the number of times of renormalization n, a temperature T' after renormalization transformation are set as the following values.

$$g' = 5.24 \times 10^6 [m/s^2]$$

$$N' = 304704$$

$$n = 20$$

$$T' = 1.05 \times 10^{20}[K] \cdots (46)$$

The gravitational acceleration g' is set so that the numbers of bonds in the granular systems before and after renormalization transformation process match each other. When the gravitational acceleration g' and the temperature T' satisfy the above-mentioned conditions, the granular system S' is in a liquid state.

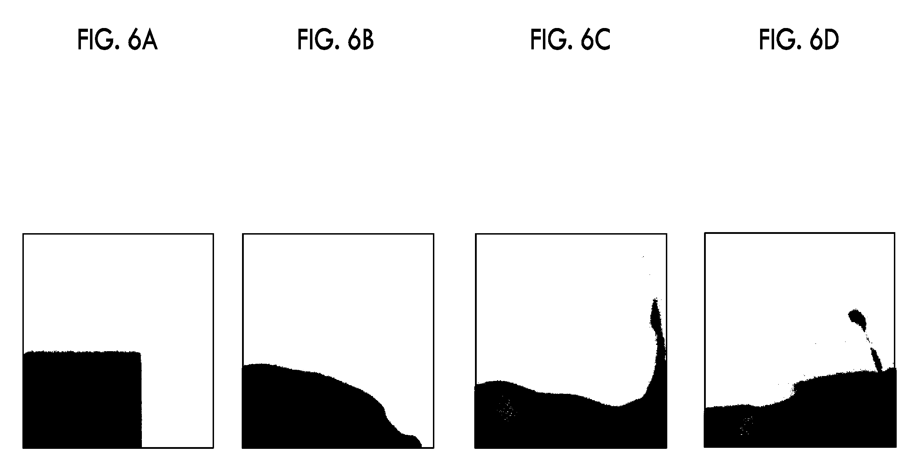

FIGS. 6A to 6D show simulation results using the simulation method according to the embodiment as figures. FIGS. 6A to 6D show cross sections vertical to the thickness direction of the dam. FIGS. 6A, 6B, 6C, and 6D show distributions of grains when t=0 [s], 0.00005 [s], 0.0001 [s], and 0.0002 [s], respectively.

If the dam is broken, as shown in FIG. 6B, a liquid flows rightward. As shown in FIG. 6C, if the liquid collides with a right wall in the figure, the liquid moves up on the wall. Then, as shown in FIG. 6D, a part of the liquid which moves up on the wall forms droplets.

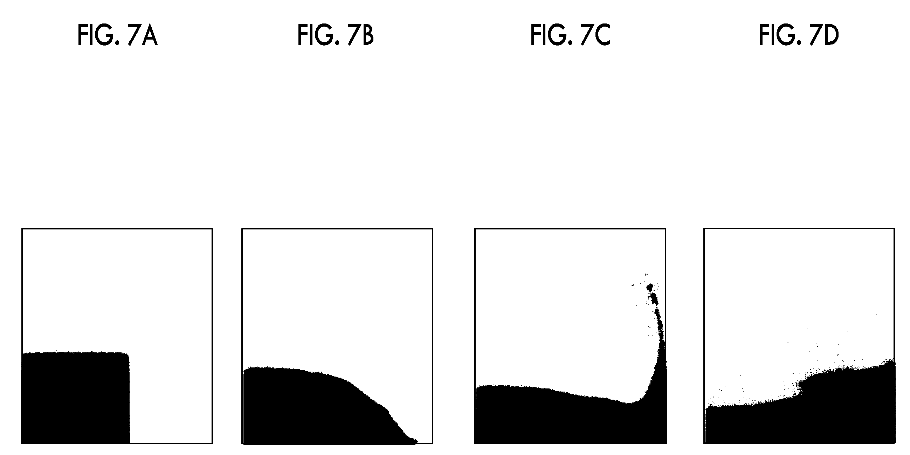

FIGS. 7A to 7D show simulation results using a simulation method according to a comparative example disclosed in Japanese Patent No. 5241468 as figures. In the comparative example, a dimension of a granular system changes according to renormalization transformation. A height H', a lateral width L', and a thickness D' of an appearance of a dam before break of a renormalized granular system S' are set as the following values, respectively.

$$H' = 50[nm]$$

$$L' = 50[nm]$$

$$D' = 5.4[nm] \cdots (47)$$

As simulation conditions, a gravitational acceleration g' after renormalization transformation, the number of grains N' after renormalization transformation, the number of times of renormalization n, a temperature T' after renormalization transformation are set as the following values.

$$g' = 5.0[m/s^2]$$

$$N' = 304704$$

$$n = 20$$

$$T' = 100[K] \cdots (48)$$

The Reynolds number and the Froude number of the simulated liquid are the same between the granular system S' renormalized by the simulation method according to the embodiment and the granular system S' renormalized by the simulation method according to the comparative example. Thus, both cases show common behaviors as fluids.

As shown in FIGS. 6A to 6D, and as shown in FIGS. 7A to 7D, in the simulation method according to the embodiment and the simulation method according to the comparative example, approximately similar results are obtained as a whole. However, in view of details, a difference therebetween is present.

In the simulation method according to the embodiment, in the state shown in FIG. 6C, Kelvin-Helmholtz instability (surface flapping phenomenon) is reproduced. On the other hand, in the simulation method according to the comparative example, in the state shown in FIG. 7C, a surface flapping phenomenon is not reproduced. Further, in the simulation method according to the embodiment, in the state shown in FIG. 6D, droplets are formed when the liquid that moves up on the wall drops. On the other hand, in the simulation method according to the comparative example, formation of droplets is not reproduced.

Form the above-described reviews, it can be understood that by applying the simulation method according to the embodiment, it is possible to correctly reproduce a behavior of a granular system.

The simulation method according to the embodiment may be realized by causing a computer to execute a computer program. The computer program may be provided in a state of being recorded on a data recording medium, for example. Alternatively, the computer program may be provided through an electric communication line.

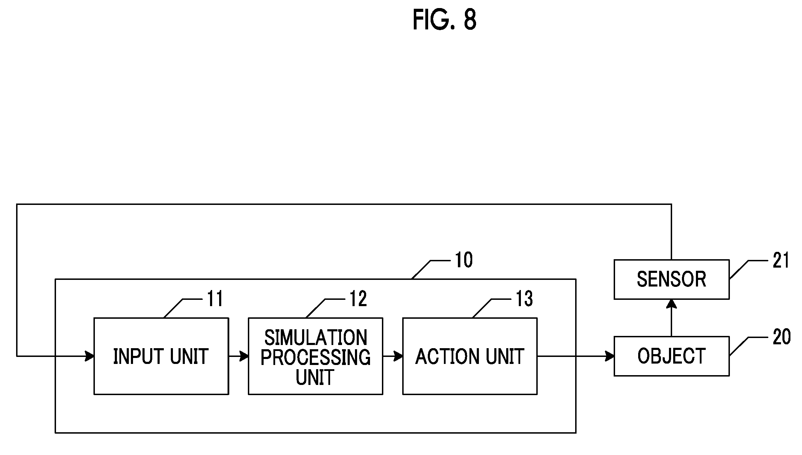

FIG. 8 is a block diagram of a simulation device 10 according to an embodiment. The simulation device 10 includes an input unit 11, a simulation processing unit 12, and an action unit 13. A sensor 21 detects various physical quantities of an object 20, for example, an appearance dimension, the amount of displacement, a temperature, or the like. Detection results of the sensor 21 are input to the input unit of the simulation device 10.

The input unit 11 converts the detection results input through the sensor 21 into data which is usable in simulation. The simulation processing unit 12 executes the simulation method shown in FIG. 1 using the data transformed by the input unit 11 as an initial condition. Thus, a future behavior of the object 20 is estimated.

The action unit 13 performs a physical action with respect to the object 20 based on the simulation result. Since the physical action is performed based on the simulation result, it is possible to perform an appropriate action with respect to the object 20.

It should be understood that the invention is not limited to the above-described embodiment, but may be modified into various forms on the basis of the spirit of the invention. Additionally, the modifications are included in the scope of the invention.

Brief Description of the Reference Symbols

10: SIMULATION DEVICE

11: INPUT UNIT

12: SIMULATION PROCESSING UNIT

13: ACTION UNIT

20: OBJECT

21: SENSOR

**Claims**

1. A simulation method comprising:

a process of performing a renormalization transformation process with respect to a granular system S which is a simulation target formed of a plurality of grains based on a renormalization factor $\alpha$ depending on the number of renormalizations; and

a process of calculating a position vector and a momentum vector of a grain of a renormalized granular system S' , by executing molecular dynamics calculation with respect to the renormalized granular system S', wherein when an interaction potential $\phi$ between the grains of the granular system S is expressed as follows,

$$\phi(r) = \varepsilon \cdot f\left(\frac{r - r_0}{\sigma}\right)$$

where $\varepsilon$ represents an interaction coefficient having a dimension of energy, f represents a non-dimensional function, $r_0$ and $\sigma$ represent parameters characterizing a grain, and r represents an inter-grain distance, and when a dimensionality of a space of the granular system S is represented as d, by applying transformation laws expressed as follows,

$$N' = \frac{N}{\alpha^d}$$

$$m' = m \cdot \alpha^d$$

$$\varepsilon' = \varepsilon \cdot \alpha^d$$

$$r_0' = \alpha \cdot r_0$$

$$\sigma' = \alpha \cdot \sigma$$

the molecular dynamics calculation is executed based on an interaction potential of the renormalized granular system S' expressed as follows:

$$\phi'(r) = \varepsilon' \cdot f\left(\frac{r - r_0'}{\sigma'}\right)$$

2. The simulation method according to claim 1, wherein when the number of renormalizations in the process of performing the renormalization transformation process is represented as n, the renormalization factor $\alpha$ is $2^n$.

3. The simulation method according to claim 1 or 2, wherein when a temperature of the granular system S is represented as T and a temperature of the renormalized granular system S' is represented as T' , initial conditions of a temperature when the molecular dynamics calculation is performed are set by applying a transformation law expressed as follows:

$$T' = T \cdot \alpha^d$$

4. A computer program that causes a computer to execute the simulation method according to any one of claims 1 to 3.

5. A recording medium on which the computer program according to claim 4 is recorded to be readable by a computer.

6. A simulation device comprising:

an input unit that converts a physical quantity detected from an object into data which is usable in a simulation;
a simulation processing unit that executes the simulation using the data converted in the input unit as an initial condition; and
an action unit that performs an action with respect to the object based on a result of the simulation executed in the simulation processing unit, wherein
when an interaction potential $\phi$ between grains of a granular system S where the object is expressed as a plurality of grains is expressed as follows,

$$\phi(r) = \varepsilon \cdot f\left(\frac{r - r_0}{\sigma}\right)$$

where $\varepsilon$ represents an interaction coefficient having a dimension of energy, f represents a non-dimensional function, $r_0$ and $\sigma$ represent parameters characterizing a grain, and r represents an inter-grain distance, the simulation processing unit executes a renormalization transformation process with respect to the granular system S where the object is expressed as the plurality of grains based on a renormalization factor $\alpha$ depending on the number of renormalizations, and executes a process of calculating a position vector and a momentum vector of a grain of a renormalized granular system S' by executing molecular dynamics calculation with respect to the renormalized granular system S', and
when a dimensionality of a space of the granular system S is represented as d,
by applying transformation laws expressed as follows,

$$N' = \frac{N}{\alpha^d}$$

$$m' = m \cdot \alpha^d$$

$$\varepsilon' = \varepsilon \cdot \alpha^d$$

$$r_0' = \alpha \cdot r_0$$

$$\sigma' = \alpha \cdot \sigma$$

the molecular dynamics calculation is executed based on an interaction potential of the renormalized granular system S' expressed as follows:

$$\phi'(r) = \varepsilon' \cdot f\left(\frac{r - r_0'}{\sigma'}\right)$$

## FIG. 1

START

↓

EXECUTE RENORMALIZATION
TRANSFORMATION PROCESS WITH
RESPECT TO GRANULAR SYSTEM S WHICH
IS SIMULATION TARGET TO DEFINE
RENORMALIZED GRANULAR SYSTEM S′ ⎯ S1

↓

SET INITIAL CONDITIONS OF SIMULATION ⎯ S2

↓

EXECUTE MOLECULAR DYNAMICS
CALCULATION WITH RESPECT TO
RENORMALIZED GRANULAR SYSTEM S′ TO
CALCULATE POSITION VECTOR q′ AND
MOMENTUM VECTOR p′ OF GRAIN ⎯ S3

↓

OUTPUT SIMULATION RESULT ⎯ S4

↓

END

# FIG. 2

FIG. 3

## FIG. 4

## FIG. 5A

● LATTICE POINT

— INTERACTION
BETWEEN NEAREST
GRAINS

## FIG. 5B

○ LATTICE POINT TO
BE ELIMINATED

FIG. 5C

FIG. 6A    FIG. 6B    FIG. 6C    FIG. 6D

FIG. 7A          FIG. 7B          FIG. 7C          FIG. 7D

EP 3 121 745 A1

# FIG. 8

Europäisches
Patentamt

European
Patent Office

Office européen
des brevets

**EUROPEAN SEARCH REPORT**

Application Number

EP 16 17 0131

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 369 514 A1 (SUMITOMO HEAVY INDUSTRIES [JP]) 28 September 2011 (2011-09-28) * the whole document * | 1-6 | INV. G06F19/00 |
| X | JP 2006 285866 A (SUMITOMO HEAVY INDUSTRIES) 19 October 2006 (2006-10-19) * the whole document * | 1-6 | |
| A | HALLEY J W ET AL: "Simulation methods for chemically specific modeling of electrochemical interfaces", ELECTROCHIMICA ACTA, ELSEVIER SCIENCE PUBLISHERS, BARKING, GB, vol. 46, no. 2-3, 1 November 2000 (2000-11-01), pages 239-245, XP004225068, ISSN: 0013-4686, DOI: 10.1016/S0013-4686(00)00578-8 * the whole document * | 1-6 | |

|  | TECHNICAL FIELDS SEARCHED (IPC) |
|---|---|
|  | G06F |

The present search report has been drawn up for all claims

| Place of search | Date of completion of the search | Examiner |
|---|---|---|
| The Hague | 15 December 2016 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 17 0131

This annex lists the patent family members relating to the patent documents cited in the above-mentioned European search report.
The members are as contained in the European Patent Office EDP file on
The European Patent Office is in no way liable for these particulars which are merely given for the purpose of information.

15-12-2016

| Patent document cited in search report | | Publication date | Patent family member(s) | | Publication date |
|---|---|---|---|---|---|
| EP 2369514 | A1 | 28-09-2011 | CN | 102257500 A | 23-11-2011 |
| | | | EP | 2369514 A1 | 28-09-2011 |
| | | | JP | 5241468 B2 | 17-07-2013 |
| | | | JP | 2010146368 A | 01-07-2010 |
| | | | KR | 20110086624 A | 28-07-2011 |
| | | | US | 2011246167 A1 | 06-10-2011 |
| | | | WO | 2010070803 A2 | 24-06-2010 |
| JP 2006285866 | A | 19-10-2006 | JP | 4666357 B2 | 06-04-2011 |
| | | | JP | 2006285866 A | 19-10-2006 |

EPO FORM P0459

For more details about this annex : see Official Journal of the European Patent Office, No. 12/82

**EP 3 121 745 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2015103371 A
- JP 524168 A
- JP 5241468 B

### Non-patent literature cited in the description

- **J.M. THIJSSEN.** Computational Physics. Cambridge University Press, 1999
- **LEO P. KADANOFF.** STATISTICAL PHYSICS Static, Dynamics and Renormalization. World Scientific, 1999